# EUROPÄISCHE PATENTSCHRIFT

(11) **EP 0 531 714 B1**
(45) Veröffentlichungstag und Bekanntmachung des Hinweises auf die Patenterteilung: **10.04.1996**
(21) Anmeldenummer: 92113460.7
(22) Anmeldetag: 07.08.1992
(51) Int. Cl.: A61F 13/15, A61F 13/56

(54) **Saugfähiges Produkt für Ausscheidungen des menschlichen Körpers**
Absorbant article for human body discharge
Article absorbant pour sécrétions du corps humain

(30) Priorität: 11.09.1991 DE 4130144
(43) Veröffentlichungstag der Anmeldung: 17.03.1993
(73) Patentinhaber: W. PELZ GMBH & CO., D-23812 Wahlstedt (DE)
(72) Erfinder: Pelz, Udo W., W-2360 Bad Segeberg (DE)
(74) Vertreter: Dipl.-Ing. H. Hauck, Dipl.-Ing. E. Graalfs, Dipl.-Ing. W. Wehnert, Dr.-Ing. W. Döring

(56) Entgegenhaltungen:
- GB-A- 2 227 174
- US-A- 4 608 047
- US-A- 4 773 905

## Beschreibung

Gegenstand der Erfindung ist ein saugfähiges Produkt für Ausscheidungen des menschlichen Körpers, insbesondere Damenbinde oder Inkontinenzprodukt, nach dem Oberbegriff des Anspruches 1.

Saugfähige Produkte vorstehender Art werden mit ihrer körperabgewandten Seite im Bereich des saugfähigen Kissens gegen das Zwickel des Höschens gelegt, worauf die Flügel um die Ränder des Zwickels gefaltet und an dessen Außenseite befestigt werden. Dies hat insbesondere den Vorteil eines gegenüber Körperbewegungen gesicherten Produktsitzes, wobei die Zwickelränder an einem Rutschen zwischen Körper und körperzugewandter Kissenseite und einhergehender Verschmutzung gehindert werden.

Eine Damenbinde der eingangs genannten Art ist aus der EP-A2-0304644 bekannt. Bei ihr wird die körperabgewandte Seite von der flüssigkeitsundurchlässigen Schicht und die körperzugewandte Seite von einer flüssigkeitsdurchlässigen Schicht gebildet. In den Flügelbereichen sind die beiden Schichten unmittelbar neben dem Kissen und an den Flügelkanten miteinander versiegelt. Enthält das Saugkissen Körperflüssigkeit, kann es durch eine Druckkraft beispielsweise infolge von Körperbewegungen des Benutzers zumindest teilweise entleert werden, wobei die Flüssigkeit über die Kissenränder austritt und ggf. über die Flügel abläuft. Dann kann die Bekleidung des Benutzers verschmutzen.

Die EP-A1-0301491 betrifft eine Damenbinde, die zwischen Kissen und Flügel eine Flüssigkeitsabdichtung hat. Die Flüssigkeitsabdichtung soll das Abströmen von Flüssigkeit aus dem Kern in die Flügelbereiche verhindern, die ebenfalls mit Absorptionsschichten für den Anfall außerordentlicher Flüssigkeitsmengen versehen sind. Die Abdichtungen stehen jedoch einem Flüssigkeitsaustritt an der körperzugewandten Seite infolge Druckeinwirkung oder stoßartigen Anfalls von Körperflüssigkeit nicht entgegen, der unmittelbar oder über die Flügel zur Verschmutzungen von Unterwäsche und Bekleidung führen kann.

Die US-A-4 773 905 bezieht sich auf eine ganz ähnliche Damenbinde, die Flüssigkeitsabdichtungen hat, welche sich quer über das Absorptionselement zwischen den beiden Flügeln erstrecken. Diese Flüssigkeitsabdichtungen sollen die Übertragung von Körperflüssigkeit von einem Zentralabschnitt des Absorptionselementes auf dessen Endabschnitte verhindern. Die Binde kann ohne Flüssigkeitsabdichtungen zwischen dem Absorptionselement und den Flügeln sein. Dann kann sich Körperflüssigkeit in einer unteren Stoffschicht aus dem Absorptionselement in die Flügel bewegen. Auch bei dieser Binde ist ein Flüssigkeitsaustritt an der körperzugewandten Seite des Absorptionselements oder an den Flügeln möglich.

Die US-A-4 608 047 bezieht sich auf eine Damenbinde mit Flügeln, die entlang einer beliebigen Linie zwischen ihrer Verbindungslinie mit dem Saugkissen und ihrem freien Ende um den Stegabschnitt eines Höschens gefaltet werden können. Dafür haben die Flügel die Faltbarkeit einer Polyethylenschicht von weniger als 0,1 mm Dicke. Austretende Körperflüssigkeit kann an den Flügeln mit Kunststoffoberfläche leicht abströmen und die Bekleidung einer Trägerin beschmutzen.

Die GB-A-2 227 174 offenbart eine Binde, deren seitliche Flügel zwecks Minderung von Leckagen in Längsrichtung versetzt sind. Die Binde kann eine flüssigkeitsundurchlässige Unterschicht, eine Absorptionsschicht auf Mittelteil und Flügeln sowie eine flüssigkeitsdurchlässige Abdeckung an der körperzugewandten Seite haben. Leckagemöglichkeiten wie bei den beiden vorbeschriebenen Binden sind also trotz der unterschiedlichen Flügelanordnung vorhanden.

Davon ausgehend liegt der Erfindung die Aufgabe zugrunde, ein saugfähiges Produkt für Ausscheidungen des menschlichen Körpers zu schaffen, das insbesondere besser gegen Flüssigkeitsabgabe infolge Druckeinwirkung oder stoßartigen Anfalls von Körperflüssigkeit gesichert ist.

Die Lösung dieser Aufgabe ist in Anspruch 1 angegeben. Vorteilhafte Ausgestaltungen sind in den Unteransprüchen zu finden.

Bei einem erfindungsgemäßen Produkt bilden die flüssigkeitsundurchlässigen Schichten in den Flügeln Taschen, die sich zum saugfähigen Kissen hin öffnen. Die Taschen können Überschußmengen an Körperflüssigkeit aufnehmen, die druckbedingt vom Kissen freigesetzt werden oder auf stoßartigem Flüssigkeitsanfall beruhen. In beiden Fällen führt der Weg des geringsten Flüssigkeitswiderstandes in die Taschen, die bevorzugt hohl ausgebildet sind und glatte Innenflächen haben. Insbesondere ein Flüssigkeitsaustritt zwischen Körper und körperzugewandter Kissenseite ist demgegenüber wegen der dort herrschenden Flächenpressung erschwert. Erfindungsgemäß wird somit die Sicherheit gegen Verschmutzung der Wäsche erheblich verbessert.

Bevorzugt bedecken die flüssigkeitsundurchlässigen Schichten die Längs-, Quer- und ggf. in Randnähe die körperzugewandten Seiten des Kissens, so daß ein Flüssigkeitsaustritt dort ebenfalls verhindert wird. Als flüssigkeitsundurchlässige Schichten kommen Polyolefine wie Polypropylen oder Polyethylen, Mischpolymere, kaschierte Vliese (z.B. Nonwoven mit Leimkaschierung) oder imprägnierte Materialien allein oder in Kombination in Betracht.

Ist die flüssigkeitsundurchlässige Schicht an der körperzugewandten Flügelseite von während der Herstellung zum Kissen hin gefalteten Abschnitten der flüssigkeitsundurchlässigen Schicht an der körperabgewandten Seite gebildet, kann die Herstellung wegen der geringeren Bestandteilezahl begünstigt sein. Ist die flüssigkeitsundurchlässige Schicht an der körperzugewandten Flügelseite von ursprünglich separaten Abschnitten gebildet, kann insbesondere eine Materialeinsparung beim Ausstanzen des flüssigkeitsundurchlässigen Schichtenmaterials erzielt werden.

Eine flüssigkeitsdurchlässige Hüllschicht an der körperzugewandten Produktseite kann Fasermaterial des Kissens zurückhalten und den Tragekomfort verbessern. Ein Klebstoffauftrag der Hüllschicht über der darunterliegenden flüssigkeitsundurchlässigen Schicht kann Flüssigkeitsleitung der Hüllschicht unterdrücken, insbesondere wenn diese aus einem Vliesmaterial besteht. Der Klebstoffauftrag ist z.B. eine Klebeverbindung oder eine Kaschierung. Die körperabgewandte Bindenseite kann von flüssigkeitsundurchlässiger Schicht gebildet sein und besteht bei einer komfortableren Version aus einer flüssigkeitsdurchlässigen Hüllschicht. Beide Hüllschichten sind randseitig mit den flüssigkeitsundurchlässigen Schichten zu verbinden.

Zwecks Lagefixierung des Saugkissens haben bevorzugt zumindest die flüssigkeitsundurchlässigen Schichten des Flügels längs des Kissens Verbindungsstellen mit zwischenliegenden Durchtrittskanälen für Körperflüssigkeit. Die Verbindungsstellen können der Kontur eines katzenzungenförmigen, d.h. im Mittelbereich eingeschnürten Kissens, folgen. Natürlich können die Verbindungsstellen auch sämtliche Hüllschichten an den Flügeln festlegen. Die flüssigkeitsdichten Verbindungsstellen können elliptisch sein. Ihre Außenkontur kann so gewählt sein, daß die Strömungsrichtung in die Taschen bevorzugt ist und Flüssigkeit dort zumindest zeitweilig gespeichert wird. Das ist bei sich zum Flügel hin verjüngenden Durchtrittskanälen der Fall und kann beispielsweise durch halbkreisförmige Verbindungsstellen erreicht werden. Der Flüssigkeitstransport in die Taschen kann durch Material mit Dochtwirkung insbesondere in den Durchtrittskanälen gefördert werden. Ferner kann in den Taschen ein absorbierendes Material wie ein Superabsorber vorhanden sein. Prägungen im Taschenbereich können ebenfalls eine Flüssigkeitsspeicherung und/oder -verteilung bewirken. Für zusätzliche Speicherung kann das flüssigkeitsundurchlässige Material auch Ausdehnungsbereiche haben, z.B. Faltenbereiche. Die Ausdehnung der Taschen kann aber auch auf einen Teil der Flügel in Quer- und/oder Längsrichtung des Produktes begrenzt sein.

Der Flüssigkeitsrückhaltung in den Taschen können außerdem Lippendichtungen dienlich sein, die im Öffnungsbereich der Taschen zum saugfähigen Kissen angeordnete Dichtlippen haben, welche ein unfreies kissennahes Ende und ein freies kissenfernes Ende in der Tasche haben. Diese Dichtlippen setzen einem Flüssigkeitsdurchtritt vom Kern in die Tasche praktisch keinen Widerstand entgegen. Sie behindern jedoch einen Rückfluß der Flüssigkeit aus den Taschen, weil dieser mit einer Druckausübung auf das freie Ende der Dichtlippen verbunden ist, welcher diese unter Versperrung des Flüssigkeitsdurchtrittes gegen eine der flüssigkeitsundurchlässigen Schichten drückt.

Das kissennahe Ende der Dichtlippen kann neben einer Öffnung der Tasche zwischen einer flüssigkeitsundurchlässigen Schicht und dem Kissen eingeklemmt sein. Bevorzugt sind die Dichtlippen jedoch an ihrem unfreien Ende mit einer flüssigkeitsundurchlässigen Schicht verbunden, wodurch bei Herstellung und Benutzung eine verbesserte Lagefixierung gegeben ist. Das kissennahe Ende kann durch Verschweißung oder Verklebung mit einer flüssigkeitsundurchlässigen Schicht auf der körperabgewandten oder der körperzugewandten Seite verbunden sein. Die flüssigkeitsundurchlässige Schicht an der körperzugewandten Seite kann aber auch mit einem zurückgefalteten kissennahen Ende eine Dichtlippe bilden. Die Dichtlippen können ebenfalls aus den zu den flüssigkeitsundurchlässigen Schichten genannten Materialien bestehen.

In jedem Öffnungsbereich der Taschen können zwei Dichtlippen angeordnet sein, von denen die eine mit der flüssigkeitsundurchlässigen Schicht an der körperzugewandten Seite und die andere mit der flüssigkeitsundurchlässigen Schicht an der körperabgewandten Seite verbunden ist. Die freibeweglichen Enden zweier benachbarter Dichtlippen dichten bei Druckausübung aneinander ab.

Weitere Einzelheiten und Vorteile des Gegenstandes der Erfindung ergeben sich aus der nachfolgenden Beschreibung der zugehörigen Zeichnungen, die vier bevorzugte Versionen zeigen. In den Zeichnungen zeigen:
- Fig. 1: eine Damenbinde mit elliptischen Verbindungsstellen der Schichten im Flügelbereich in der Draufsicht;
- Fig. 2: dieselbe Damenbinde in einem zentralen Querschnitt;
- Fig. 3: eine Damenbinde mit halbkreisförmigen Verbindungsstellen im Flügelbereich in der Draufsicht;
- Fig. 4: dieselbe Damenbinde in einem zentralen Querschnitt;
- Fig. 5: Damenbinde mit Haftkleber zwischen flüssigkeitsdurchlässiger Hülle und flüssigkeitsundurchlässiger Schicht im zentralen Querschnitt;
- Fig. 6: Inkontinenzprodukt mit über die Enden des Saugkissens gelegter flüssigkeitsundurchlässiger Schicht in der Draufsicht;
- Fig. 7: dasselbe Inkontinenzprodukt im zentralen Querschnitt;
- Fig. 8: eine Damenbinde mit jeweils zwei Dichtlippen an jeder Seite in der Draufsicht;
- Fig. 9: dieselbe Damenbinde im zentralen Querschnitt;
- Fig. 10: eine Damenbinde mit flüssigkeitsdichter Leimschicht an der körperzugewandten Seite und nur einer Dichtlippe in jedem Flügel im zentralen Querschnitt;
- Fig. 11: eine Damenbinde mit flüssigkeitsundurchlässiger Folie an der körperzugewandten Seite und nur einer Dichtlippe in jeder Tasche im zentralen Querschnitt;
- Fig. 12: eine Damenbinde mit verkleinerten Taschen und jeweils einer Dichtlippe in den Taschen im zentralen Querschnitt.

Nachfolgend werden die einander entsprechenden Bestandteile der verschiedenen Produkte mit übereinstimmenden Bezugsziffern bezeichnet.

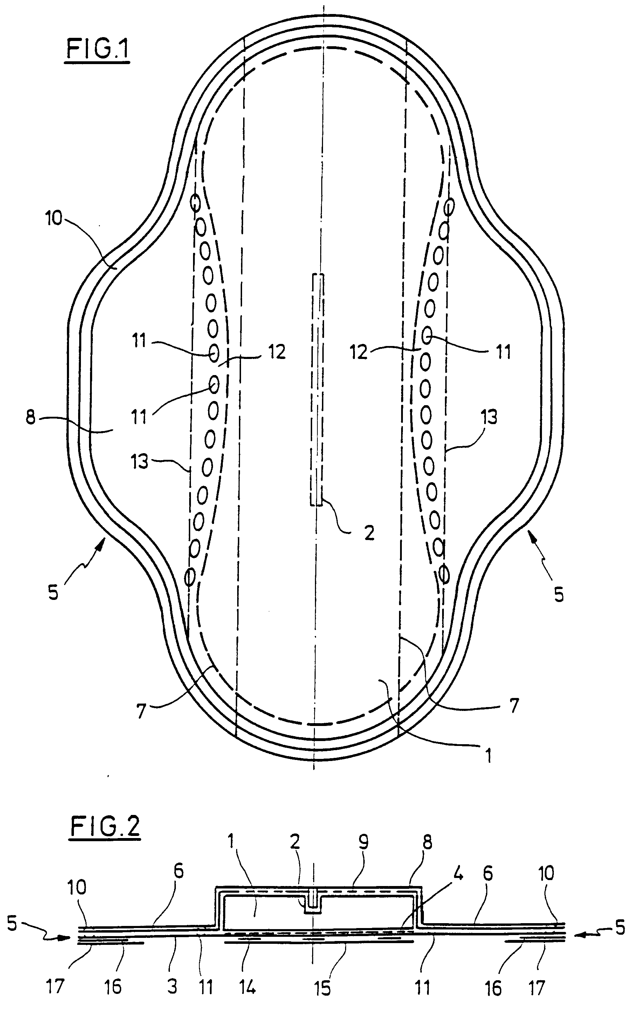

Die Damenbinde gemäß Fig. 1 und 2 hat ein Saugkissen 1, das insbesondere aus Gründen des Tragekomforts eine Katzenzungenform, d.h. eine Taille im Mittelbereich, aufweist. An der körperzugewandten Seite ist das Saugkissen 1 mit einer Kanalprägung 2 versehen, die das Einströmen von Körperflüssigkeit unterstützt.

An der körperabgewandten Kissenseite ist als flüssigkeitsundurchlässige Schicht eine Wäscheschutzfolie 3 aus Polyolefin angeordnet. Die Wäscheschutzfolie 3 ist mittels eines Schuppenleimauftrages 4 von 2 bis 4 g/m² an dem Saugkissen 1 befestigt. Ihre Kontur entspricht in den Endbereichen der Binde etwa derjenigen des Saugkissens 1 und umgrenzt an dessen Längsseiten Flügel 5.

"Körperzugewandt" und "körperabgewandt" beziehen sich in dieser Anmeldung hinsichtlich der Flügel auf deren ausgebreitete Anordnung gemäß Fig. 2. Auf der körperzugewandten Seite haben die Flügel 5 streifenförmige Wäscheschutzfolien 6, die eine körperzugewandte flüssigkeitsundurchlässige Schicht bilden und ebenfalls aus Polyäthylen bestehen. Sie sind über die Seitenkanten des Saugkissens 1 gelegt und über dessen körperzugewandte Ränder gefaltet. Der Mittelbereich des Saugkissens 1 bleibt von Wäscheschutzfolie 6 unbedeckt. Eine Überdeckung des Saugkissens 1 durch Wäscheschutzfolie 6 ist an dessen Längsseiten bis zu den Begrenzungslinien 7 gegeben.

Die körperzugewandte Bindenseite ist von einem hydrophoben Nonwoven-Vlies 8 bedeckt, welches eine flüssigkeitsdurchlässige Hüllschicht bildet. Zur Befestigung auf dem Saugkissen 1 hat es einen Schuppenleimauftrag 9 von 2 bis 4 g/m² bzw. vorzugsweise 20 g/m² im Bereich der Kanalprägung 2. Das Vlies 8 überdeckt die Seiten des Saugkissens 1, folgt seiner Kontur an den Enden und erstreckt sich bis in die Flügel 5 hinein. Das Vlies 8 sowie die Wäscheschutzfolien 6 und 3 sind randseitig mittels einer umlaufenden Versiegelung 10 oder Verschweißung miteinander verbunden. Die Versiegelung 10 dichtet den zwischen Wäscheschutzfolien 3 und 6 vorhandenen Zwischenraum zu den Randseiten des Erzeugnisses hin ab.

Neben dem Saugkissen 1 sind Vlies 8, Wäscheschutzfolien 6 und 3 über elliptische Verbindungsstellen 11 miteinander verbunden, deren Hauptachsen etwa parallel zur Kontur des Saugkissens 1 ausgerichtet sind. Die Verbindungsstellen fixieren das Saugkissen 1 in den dieses umgebenden Schichten, wobei sie die Wäscheschutzfolien 3 und 6 bereichsweise flüssigkeitsdicht miteinander verbinden und das Vlies 8 in Kissennähe festlegen. Zwischen den Verbindungsstellen 11 sind Durchtrittskanäle 12 ausgebildet, welche ein Überströmen von Flüssigkeit aus dem Saugkissen 1 in die Flügel 5 ermöglichen.

Die Flügel 5 sind über Faltlinien 13 klappbar, damit sie um den Zwickel eines Höschens gelegt werden können. An der körperabgewandten Seite trägt die Wäscheschutzfolie 3 eine zentrale Gruppe von Haftklebestreifen 14 mit einer zentralen Silikonpapierabdeckung 15 zur mittigen Festlegung der Binde am Zwickel. Die körperabgewandten Seiten der Flügel 5 sind in Randnähe mit randseitigen Haftklebestreifen 16 und zugeordneten Silikonpapierabdeckungen 17 versehen.

Nach Entfernen der Silikonpapierabdeckungen 15, 17 kann die Binde lösbar am Höschen festgeklebt werden.

Insbesondere aus Gründen der optischen Gefälligkeit und der Herstellung ist die Binde wie auch die nachfolgenden bezüglich ihrer Längs- und Querachsen symmetrisch ausgebildet.

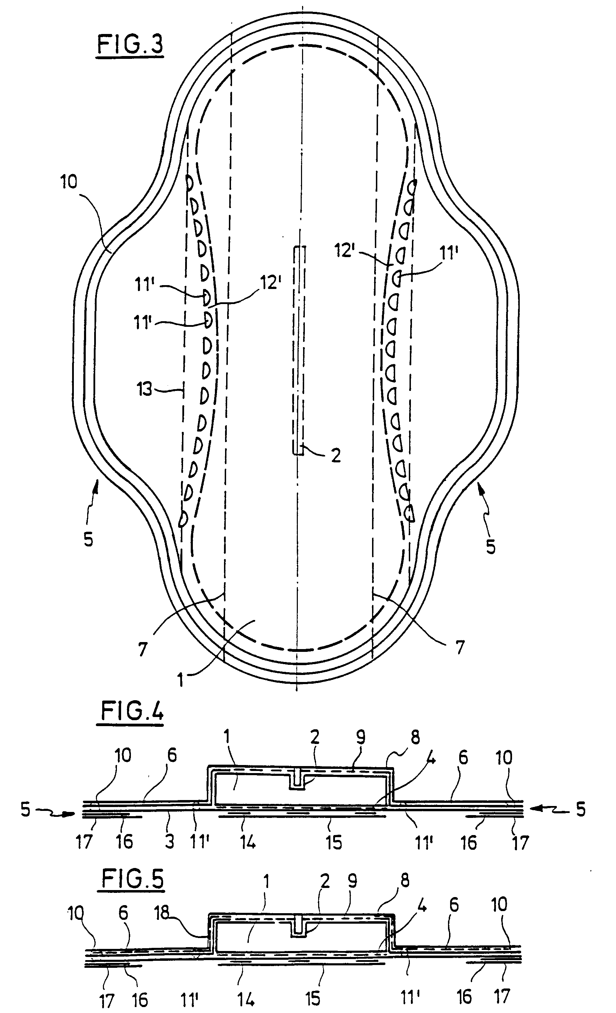

Die Damenbinde gemäß Fig. 3 und 4 unterscheidet sich von der zuvor beschriebenen dadurch, daß die Verbindungsstellen 11' anstatt einer Ellipsenform eine Halbkreisform aufweisen. Die Sehnen der Halbkreise 11' sind vom Saugkissen 1 weg- und parallel zu dessen Kontur gerichtet, so daß die zwischenliegenden Durchtrittskanäle 12' in Richtung vom Kissen 1 in die Taschen 5 einen geringeren Strömungswiderstand haben als umgekehrt. Infolgedessen wird aus dem Kissen gedrückte oder stoßartig anfallende Flüssigkeit, wie Menstruationsflüssigkeit, zumindest zeitweilig in den Flügeln gespeichert und vorzugsweise dort festgehalten. Insgesamt weist die Binde somit eine erhöhte Aufnahmekapazität auf. Im übrigen treffen die Erläuterungen zu den Fig. 1 und 2 auch für diese Damenbinde zu.

Die Damenbinde gemäß Fig. 5 haben gegenüber den zuvor beschriebenen zwischen dem Vlies 8 und den beiden streifenförmigen Wäscheschutzfolien 6 der körperzugewandten Bindenseite einen Kleber 18, der als Schuppenleimauftrag ausgebildet ist. Der Kleber 18 sorgt für eine flächige Festlegung des non-wovens 8 an den Wäscheschutzfolien 6 und wirkt einer Flüssigkeitsleitung durch das non-woven entgegen, insbesondere wenn es aus hydrophilem Material besteht.

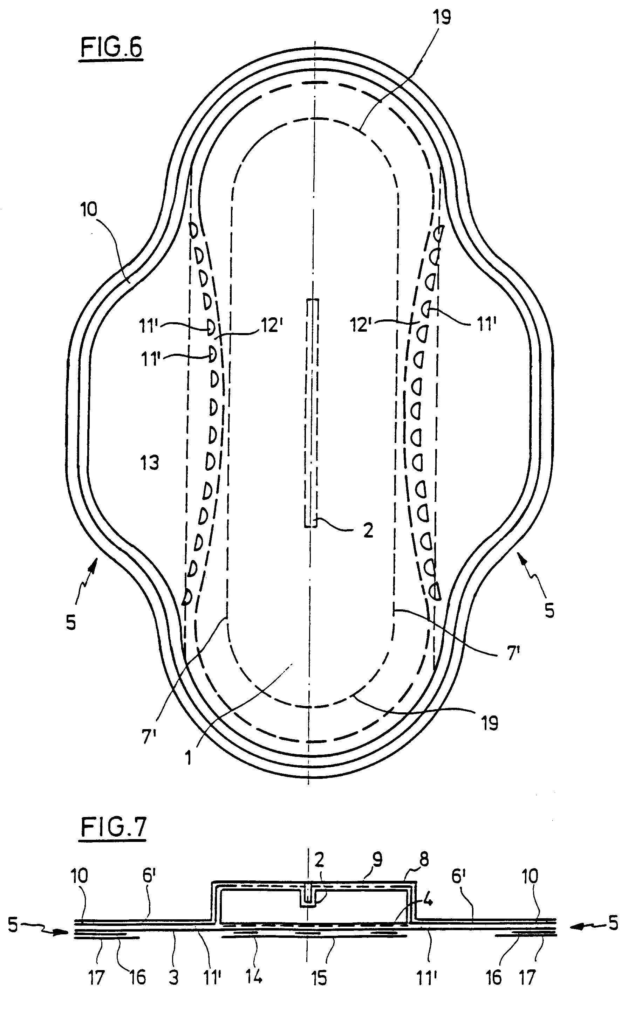

Das Inkontinenzprodukt nach den Fig. 6 und 7 unterscheidet sich von der Damenbinde gemäß Fig. 3 und 4 dadurch, daß die Wäscheschutzfolien 6' das Saugkissen 1 an der körperzugewandten Seite nicht nur mit einem Längsrand 7', sondern auch mit Querrändern 19 überlappen, die der gekrümmten Kissenkontur folgen. Die Wäscheschutzfolien 6' erstrecken sich von den Querrändern 19 aus ebenfalls über die Seiten des Saugkissens 1 bis in die endseitig vorstehenden Ränder hinein, in denen sie mit dem Non-woven-Vlies 8 und der Wäscheschutzfolie 3 der körperabgewandten Seite über die Versiegelung 10 verbunden sind. Die Wäscheschutzfolie 6' kann in diesem Fall vor der Verbindung mit den übrigen Bestandteilen des Kissens auch einteilig ausgestanzt sein. Insgesamt wird hierdurch eine weiter verbesserte Flüssigkeitsabdichtung erzielt.

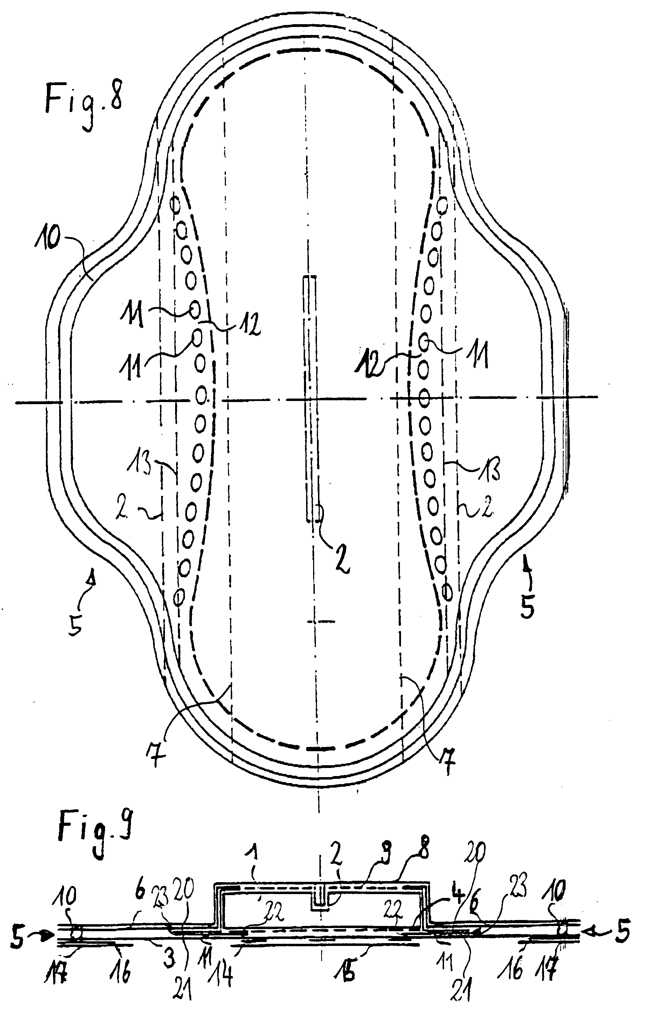

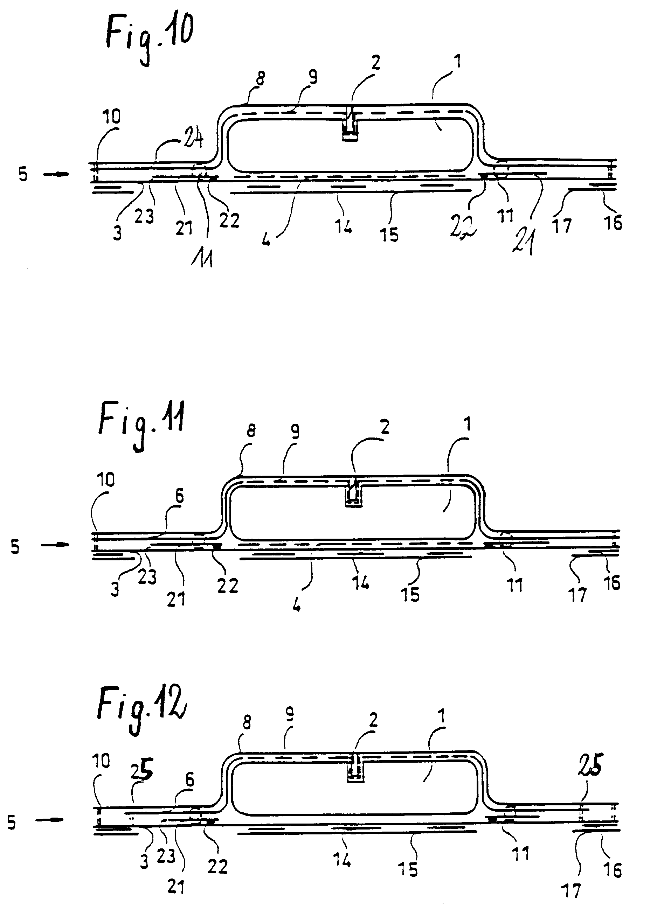

Die Damenbinde nach den Fig. 8 und 9 hat an der körperzugewandten Seite streifenförmige Wäscheschutzfolien 6, die von den Begrenzungslinien 7 auf dem Kissen 1 nach innen zurückgefaltet sind, wobei sie bis in die Taschen der Flügel 5 hineinreichen und dort Dichtlippen 20 bilden. Es sind weitere Dichtlippen 21 vorgesehen, die zwischen Kissen 1 und flüssigkeitsundurchlässiger Schicht 3 an der körperabgewandten Seite jeweils eine kissennahe Verschweißung 22 mit letzterer Schicht haben. Beide Dichtlippen 20, 21 erstrecken sich in Längsrichtung der Binde über den gesamten Öffnungsbereich des Kissens 1 zum jeweiligen Flügel 5. Zusätzlich werden sie dabei von den Verbindungsstellen 11 fixiert, wobei die Durchtrittskanäle 12 zwischen den Dichtlippen 20, 21 verlaufen.

Ausgehend vom Kissen 1 kann Flüssigkeit praktisch unbehindert zwischen den Dichtlippen 20, 21 hindurch in die Taschen der Flügel 5 gelangen. Eine Druckerhöhung in den Taschen der Flügel 5 führt dazu, daß sich die freien Enden der Dichtlippen 20, 21 bei 23 aneinanderlegen und eine Rückströmung von Flüssigkeit verhindern.

Im Unterschied zu dieser Ausführungsform ist gemäß Fig. 10 nur eine Dichtlippe 21 in jeder Tasche vorgesehen und praktisch im Öffnungsquerschnitt zwischen Kissen 1 und Flügeln 5 mit der flüssigkeitsundurchlässigen Folie an der körperabgewandten Seite über eine Verschweißung 22 verbunden. Außerdem ist statt einer Folie 6 an der körperzugewandten Seite eine flüssigkeitsdichte Leimschicht 24 vorgesehen, die auf dem Nonwoven-Vlies 8 der körperzugewandten Seite aufgetragen ist. Das freie Ende 23 der Dichtlippen 21 kann sich bei Druckerhöhung in den Taschen an die Leimschicht 24 anlegen und einen Rückfluß von Flüssigkeit verhindern.

Die Leimschicht kann ein Flächengewicht von 35 bis 70 g/m² haben.

Im Unterschied dazu ist bei der Version gemäß Fig. 11 keine Leimschicht, sondern eine Folie 6 an der Innenseite des körperzugewandten Vlieses angeordnet. Die Dichtlippe 21 dichtet mit ihrem freien Ende 23 gegenüber der Folie 6 ab.

Die Damenbinde gemäß Fig. 12 hat zwar ebenfalls eine Folie 6 an der körperzugewandten Seite. Die Folie 6 erstreckt sich jedoch in Querrichtung der Damenbinde nicht über die gesamte Breite der Flügel 5, sondern ist über eine zusätzliche Schweiß- oder Klebenaht 25 randseitig abgedichtet.

## Patentansprüche

1. Saugfähiges Produkt für Ausscheidungen des menschlichen Körpers, insbesondere Damenbinde oder Inkontinenzprodukt, mit einem saugfähigen Kissen, seitlich des Kissens angeordneten Flügeln mit einem Flüssigkeitsdurchgang zwischen Kissen und Flügeln und einer flüssigkeitsundurchlässigen Schicht an der körperabgewandten Seite, dadurch gekennzeichnet, daß eine weitere flüssigkeitsundurchlässige Schicht (6) an der körperzugewandten Seite der Flügel (5) angeordnet und randseitig flüssigkeitsdicht mit der flüssigkeitsundurchlässigen Schicht (3) an der körperabgewandten Seite verbunden ist und die von den flüssigkeitsundurchlässigen Schichten (3, 6) in den Flügeln (5) gebildeten Taschen zum saugfähigen Kissen (1) hin geöffnet sind.

2. Produkt nach Anspruch 1, dadurch gekennzeichnet, daß die flüssigkeitsundurchlässigen Schichten (6) die Längsseiten des Kissens (1) bedecken.

3. Produkt nach Anspruch 1 oder 2, dadurch gekennzeichnet, daß die flüssigkeitsundurchlässigen Schichten (6') die Querseiten des Kissens (1) bedecken.

4. Produkt nach einem der Ansprüche 1 bis 3, dadurch gekennzeichnet, daß die flüssigkeitsundurchlässigen Schichten (6, 6') die körperzugewandte Seite des Kissens (1) randseitig bedecken.

5. Produkt nach einem der Ansprüche 1 bis 4, dadurch gekennzeichnet, daß die flüssigkeitsundurchlässigen Schichten an den körperzugewandten Seiten der Flügel von zurückgefalteten Abschnitten der flüssigkeitsundurchlässigen Schicht an der körperabgewandten Seite gebildet sind.

6. Produkt nach einem der Ansprüche 1 bis 4, dadurch gekennzeichnet, daß die flüssigkeitsundurchlässigen Schichten (6) an der körperzugewandten Seite der Flügel (5) von vor randseitig flüssigkeitsdichter Verbindung von der flüssigkeitsundurchlässigen Schicht (3) an der körperabgewandten Seite getrennten Abschnitten gebildet sind.

7. Produkt nach einem der Ansprüche 1 bis 6, dadurch gekennzeichnet, daß die körperzugewandte Seite eine flüssigkeitsdurchlässige Hüllschicht (8) hat.

8. Produkt nach Anspruch 7, dadurch gekennzeichnet, daß die Hüllschicht (8) über der körperzugewandten flüssigkeitsundurchlässigen Schicht (6) einen Klebstoffauftrag (18) hat.

9. Produkt nach einem der Ansprüche 1 bis 8, dadurch gekennzeichnet, daß die körperabgewandte Seite eine flüssigkeitsdurchlässige Hüllschicht hat, die randseitig mit der Hüllschicht an der körperzugewandten Seite verbunden ist.

10. Produkt nach einem der Ansprüche 1 bis 9, dadurch gekennzeichnet, daß zumindest die flüssigkeitsundurchlässigen Schichten (3, 6) der Flügel (5) längs des Kissens (1) Verbindungsstellen (11) mit zwischenliegenden Durchtrittskanälen (12) zwischen Kissen (1) und Taschen für Körperflüssigkeit haben.

11. Produkt nach Anspruch 10, dadurch gekennzeichnet, daß die Verbindungsstellen (11) elliptisch mit längs der Kontur des Kissens (1) ausgerichteten Hauptachsen sind.

12. Produkt nach Anspruch 10, dadurch gekennzeichnet, daß die Durchtrittskanäle (12') in Richtung vom Kissen (1) in die Taschen einen geringeren Strömungswiderstand als umgekehrt haben.

13. Produkt nach Anspruch 12, dadurch gekennzeichnet, daß die Verbindungsstellen (11') halbkreisförmig sind und vom Kissen (1) abgewandte und zu dessen Kontur parallele Kreissehnen haben.

14. Produkt nach einem der Ansprüche 1 bis 13, dadurch gekennzeichnet, daß der Flüssigkeitseintritt in die Taschen durch ein Material mit Dochtwirkung gefördert wird.

15. Produkt nach einem der Ansprüche 1 bis 14, dadurch gekennzeichnet, daß die Taschen ein absorbierendes Material enthalten.

16. Produkt nach einem der Ansprüche 1 bis 15, dadurch gekennzeichnet, daß die flüssigkeitsundurchlässigen Schichten in den Taschen Prägungen für eine Flüssigkeitsspeicherung und/oder Verteilung haben.

17. Produkt nach einem der Ansprüche 1 bis 16, dadurch gekennzeichnet, daß das flüssigkeitsundurchlässige Material in den Taschen Ausdehnungsbereiche hat.

18. Produkt nach einem der Ansprüche 1 bis 17, dadurch gekennzeichnet, daß die Taschen nur in einem Teil der Flügel (5) ausgebildet sind.

19. Produkt nach einem der Ansprüche 1 bis 18, dadurch gekennzeichnet, daß im Öffnungsbereich der Taschen zum saugfähigen Kissen (1) Dichtlippen (20, 21) angeordnet sind, die ein unfreies kissennahes Ende und ein freies kissenfernes Ende in der Tasche haben.

20. Produkt nach Anspruch 19, dadurch gekennzeichnet, daß die Dichtlippen (20, 21) an ihrem unfreien Ende mit einer flüssigkeitsundurchlässigen Schicht (3, 6) verbunden sind.

21. Produkt nach Anspruch 19 oder 20, dadurch gekennzeichnet, daß Dichtlippen (21) an ihrem kissennahen Ende durch Verschweißung oder Verklebung (22) mit einer flüssigkeitsundurchlässigen Schicht (3) verbunden sind.

22. Produkt nach einem der Ansprüche 19 bis 21, dadurch gekennzeichnet, daß die flüssigkeitsundurchlässigen Schichten (6) an der körperzugewandten Seite mit einem zurückgefalteten kissennahen Ende eine Dichtlippe (20) bilden.

23. Produkt nach einem der Ansprüche 19 bis 22, dadurch gekennzeichnet, daß in jedem Öffnungsbereich zwei Dichtlippen (20, 21) angeordnet sind, von denen die eine (20) mit der flüssigkeitsundurchlässigen Schicht (6) an der körperzugewandten Seite und die andere mit der flüssigkeitsundurchlässigen Schicht (3) an der körperabgewandten Seite verbunden ist.

## Claims

1. An absorbent article for human body discharge, especially a sanitary towel or incontinence product, which comprises an absorbent padding, wings being provided at the side of said padding and including a liquid passage between said padding and said wings, as well as a layer being impermeable to liquids and provided on the side opposite to the human body, characterized in that a further layer (6) being impermeable to liquids is arranged on that side of said wings (5) which faces the human body and is connected to said layer (3) being impermeable to liquids and provided on the side opposite to the human body so as to be tight to liquids edge-sided, and that the pockets formed in said wings (5) by said layers (3, 6) being impermeable to liquids are open towards said absorbent padding (1).

2. The product according to claim 1, characterized in that said layers (6) being impermeable to liquids cover the long sides of said padding (1).

3. The product according to claim 1 or 2, characterized in that said layers (6') being impermeable to liquids cover the cross sides of said padding (1).

4. The product according to any of the claims 1 to 3, characterized in that said layers (6, 6') being impermeable to liquids cover that side of said padding (1) facing the human body on the side of the edges.

5. The product according to any of the claims 1 to 4, characterized in that, on the sides of said wings facing the human body, said layers being impermeable to liquids are formed by those portions of the layer being impermeable to liquids which are folded back on the side opposite to the human body.

6. The product according to any of the claims 1 to 4, characterized in that, on the side of said wings (5) facing the human body, said layers (6) being impermeable to liquids are formed by portions separated from the layer (3) being impermeable to liquids on the side opposite to the human body before making the edge-sided connection being tight to liquids.

7. The product according to any of the claims 1 to 6, characterized in that the side facing the human body comprises an enveloping layer (8) being permeable to liquids.

8. The product according to claim 7, characterized in that over said layer (6) being impermeable to liquids and facing the human body said enveloping layer (8) includes an adhesive layer (18).

9. The product according to any of the claims 1 to 8, characterized in that the side opposite to the human body comprises an enveloping layer being permeable to liquids which, edge-sided, is connected to said enveloping layer on the side facing the human body.

10. The product according to any of the claims 1 to 9, characterized in that at least said liquid proof layers (3, 6) of said wings (5) include junction points (11) along said padding (1) which have passages (12) therebetween between said padding (1) and said pockets for human body discharge.

11. The product according to claim 10, characterized in that said junction points (11) are elliptical and include main axes being aligned along the contour of said padding (1).

12. The product according to claim 10, characterized in that from said padding (1) towards said pockets said passages (12') have a lower flow resistance than vice versa.

13. The product according to claim 12, characterized in that said junction points (11') are semicircular and comprise chords provided opposite to said padding (1) and parallel to the contour thereof.

14. The product according to any of the claims 1 to 13, characterized in that the liquid entrance into said pockets is expedited by a material having a wick effect.

15. The product according to any of the claims 1 to 14, characterized in that said pockets include an absorbent material.

16. The product according to any of the claims 1 to 15, characterized in that said layers being impermeable to liquids comprise embossings in said pockets for storing and/or distributing the liquid.

17. The product according to any of the claims 1 to 16, characterized in that said material being impermeable to liquids includes expansion portions in said pockets.

18. The product according to any of the claims 1 to 17, characterized in that said pockets are defined in one part of said wings (5) only.

19. The product according to any of the claims 1 to 18, characterized in that within the opening area of said pockets towards said absorbent padding (1) sealing lips (20, 21) are arranged which within the pocket have one end not being free and provided adjacent to said padding, as well as one free end provided at a distance from said padding.

20. The product according to claim 19, characterized in that at their end not being free said sealing lips (20, 21) are connected to a layer (3, 6) being impermeable to liquids.

21. The product according to claim 19 or 20, characterized in that at their end provided adjacent to said padding said sealing lips (21) are connected to a layer (3) being impermeable to liquids by means of welding or bonding (22).

22. The product according to any of the claims 19 to 21, characterized in that on the side facing the human body said layers (6) being impermeable to liquids form a sealing lip (20) together with an end provided adjacent to said padding and being folded back.

23. The product according to any of the claims 19 to 22, characterized in that within each opening area two sealing lips (20, 21) are provided, one (20) of which is connected to said layer (6) being impermeable to liquids on the side facing the human body, while the other one is connected to said layer (3) being impermeable to liquids on the side opposite to the human body.

## Revendications

1. Article absorbant des sécrétions du corps humain, en particulier serviette hygiénique ou article pour incontinents, comportant un coussin absorbant, des ailes disposées sur les côtés du coussin, comportant un passage pour liquides entre le coussin et les ailes, et une couche imperméable aux liquides située du côté non tourné vers le corps, caractérisé en ce qu'une autre couche imperméable aux liquides (6) est disposée du côté de l'aile (5) qui est tourné vers le corps et, sur le bord, est reliée de manière étanche aux liquides à la couche imperméable aux liquides (3), du côté non tourné vers le corps, et en ce que les poches formées dans les ailes (5) par les couches imperméables aux liquides (3, 6) sont ouvertes en direction du coussin absorbant (1).

2. Article selon la revendication 1, caractérisé en ce que les couches imperméables aux liquides (6) recouvrent les côtés longitudinaux du coussin (1).

3. Article selon la revendication 1 ou 2, caractérisé en ce que les couches imperméables aux liquides (6') recouvrent les côtés transversaux du coussin (1).

4. Article selon l'une quelconque des revendications 1 à 3, caractérisé en ce que les couches imperméables aux liquides (6, 6') recouvrent le bord du côté du coussin (1) qui est tourné vers le corps.

5. Article selon l'une quelconque des revendications 1 à 4, caractérisé en ce que les couches imperméables aux liquides situées sur le côté des ailes qui est tourné vers le corps sont formées par des tronçons repliés de la couche imperméable aux liquides située du côté non tourné vers le corps.

6. Article selon l'une quelconque des revendications 1 à 4, caractérisé en ce que les couches imperméables aux liquides (6), situées du côté des ailes (5) qui est tourné vers le corps, sont formées par des parties séparées, en vue d'une liaison étanche aux liquides entre les bords et la couche imperméable aux liquides (3) située sur le côté non tourné vers le corps.

7. Article selon l'une quelconque des revendications 1 à 6, caractérisé en ce que le côté tourné vers le corps présente une couche d'enveloppe perméable aux liquides (8).

8. Article selon la revendication 7, caractérisé en ce que la couche d'enveloppe (8) présente une couche de colle (18) au-dessus de la couche imperméable aux liquides (6) qui est tournée vers le corps.

9. Article selon l'une quelconque des revendications 1 à 8, caractérisé en ce que le côté non tourné vers le corps présente une couche d'enveloppe perméable aux liquides, qui est reliée au bord de la couche d'enveloppe située du côté tourné vers le corps.

10. Article selon l'une quelconque des revendications 1 à 9, caractérisé en ce qu'au moins les couches imperméables aux liquides (3, 6) des ailes (5) présentent le long du coussin (1) des points de liaison (11) avec des canaux de traversée (12) situés entre le coussin (1) et les poches à liquide corporel.

11. Article selon la revendication 10, caractérisé en ce que les points de liaison (11) sont elliptiques et présentent un grand axe orienté suivant le contour du coussin (1).

12. Article selon la revendication 10, caractérisé en ce que, dans la direction allant du coussin (1) vers les poches, les canaux de traversée (12') présentent une résistance à l'écoulement plus faible que dans la direction opposée.

13. Article selon la revendication 12, caractérisé en ce que les points de liaison (11') sont en forme de demi-cercle dont les cordes sont situées du côté opposé au coussin (1) et sont parallèles au contour de celui-ci.

14. Article selon l'une quelconque des revendications 1 à 13, caractérisé en ce que la pénétration de liquides dans les poches est favorisée par un matériau présentant un effet de mèche.

15. Article selon l'une quelconque des revendications 1 à 14, caractérisé en ce que les poches contiennent un matériau absorbant.

16. Article selon l'une quelconque des revendications 1 à 15, caractérisé en ce que les couches imperméables aux liquides présentent dans les poches des creux en vue d'une accumulation et/ou une distribution de liquides.

17. Article selon l'une quelconque des revendications 1 à 16, caractérisé en ce que le matériau imperméable aux liquides présente dans les poches des régions extensibles.

18. Article selon l'une quelconque des revendications 1 à 17, caractérisé en ce que les poches sont formées uniquement dans une partie des ailes (5).

19. Article selon l'une quelconque des revendications 1 à 18, caractérisé en ce que dans la région de l'ouverture des poches vers le coussin absorbant (1) sont disposées des lèvres d'étanchéité (20, 21) qui présentent du côté du coussin une extrémité non libre et dans les poches une extrémité libre éloignée du coussin.

20. Article selon la revendication 19, caractérisé en ce que, par leur extrémité non libre, les lèvres d' étanchéité (20, 21) sont reliées à une couche imperméable aux liquides (3, 6).

21. Article selon la revendication 19 ou 20, caractérisé en ce qu'à leur extrémité proche du coussin, des lèvres d'étanchéité (21) sont reliées par soudage ou collage (22) à une couche imperméable aux liquides (3).

22. Article selon l'une quelconque des revendications 19 à 21, caractérisé en ce que les couches imperméables aux liquides (6) forment du côté tourné vers le corps une lèvre d'étanchéité (20) présentant une extrémité repliée proche du coussin.

23. Article selon l'une quelconque des revendications 19 à 22, caractérisé en ce que dans chaque région d'ouverture sont disposées deux lèvres d'étanchéité (20, 21) dont l'une (20) est reliée au côté tourné vers le corps par la couche imperméable aux liquides (6), et dont l'autre est reliée au côté non tourné vers le corps par la couche imperméable aux liquides (3).
